# EUROPÄISCHE PATENTSCHRIFT

(11) **EP 1 524 988 B1**
(45) Veröffentlichungstag und Bekanntmachung des Hinweises auf die Patenterteilung: **16.04.2008**
(21) Anmeldenummer: 03771101.7
(22) Anmeldetag: 25.07.2003
(51) Int. Cl.: A61K 38/00, A61K 38/06, A61K 38/57

(54) **VERWENDUNG EINES PROTEASOM-INHIBITORS ZUR BEHANDLUNG FIBROTISCHER ERKRANKUNGEN**
USE OF A PROTEASOME INHIBITOR IN THE TREATMENT OF FIBROTIC DISEASES
UTILISATION D'UN INHIBITEUR DU PROTEASOME POUR LE TRAITEMENT DE MALADIES FIBROTIQUES

(30) Priorität: 25.07.2002 DE 10233929
(43) Veröffentlichungstag der Anmeldung: 27.04.2005
(73) Patentinhaber: Charité-Universitätsmedizin Berlin, 10117 Berlin (DE)
(72) Erfinder: STANGL, Karl, 10709 Berlin (DE); MEINERS, Sike, 10555 Berlin (DE); HOCHER, Berthold, 14532 Kleinmachnow (DE)
(74) Vertreter: Krauss, Jan
(86) Internationale Anmeldenummer: PCT/EP2003/008205
(87) Internationale Veröffentlichungsnummer: WO 2004/011019

(56) Entgegenhaltungen:
- WO-A-02/12196
- WO-A-98/08825
- US-B1- 6 255 453
- DATABASE BIOSIS [Online] BIOSCIENCES INFORMATION SERVICE, PHILADELPHIA, PA, US; Juni 2001 (2001-06) OKUNO MASATAKA ET AL: "Prevention of rat hepatic fibrosis by the protease inhibitor, camostat mesilate, via reduced generation of active TGF-beta" Database accession no. PREV200100309222 XP002262872 & GASTROENTEROLOGY, Bd. 120, Nr. 7, Juni 2001 (2001-06), Seiten 1784-1800, ISSN: 0016-5085

## Beschreibung

Die vorliegende Erfindung bezieht sich auf die Verwendung mindestens eines Proteasom-Inhibitors zur Behandlung fibrotischer Erkrankungen insbesondere des kardiovaskulären Systems.

Die Myokardfibrose ist eine Antwort auf die Überlastung des Herzmuskels, welche beispielsweise durch Bluthochdruck, Herzinfarkt oder Kardiomyopathien verursacht wird. Zu diesem Phänomen tragen zahlreiche pathophysiologische Mechanismen bei, wie etwa die Proliferation von Herzbindegewebszellen, die zu einer erhöhten Matrixbildung führen. Funktionelle Endzustände sind die Massenzunahme des Herzmuskels und ein verstärkter bindegewebiger Umbau (Fibrose). Ein Kennzeichen des Umbaus am Herzen ist somit die interstitielle Fibrose, welche zu einer erhöhten Steifheit der Herzwände führt. Dies mündet funktionell in eine diastolische Dysfunktion, die die Herzmuskelschwäche weiter verschlimmern kann. Teilweise werden diese Effekte durch eine verstärkte Expression von Genen verursacht, die für extrazelluläre Matrixproteine, wie Collagen I und III, die dominanten Kollagene des Herzmuskels, kodieren.

Die zur Zeit am besten etablierte Strategie zur Verringerung der Herzfibrose besteht in der pharmakologischen Inhibierung des Renin-Angiotensin-Systems, beispielsweise durch ACE-Inhibitoren oder Angiotensin II-Blocker. Diese Substanzen haben jedoch den Nachteil, dass sie in wenigstens 5% der Fälle zu Unverträglichkeiten bei den Patienten führen. Weiterhin wirken sie nur auf die durch das Renin-Angiotensin-System vermittelte Herzfibrose und nicht auf die durch andere Agonisten wie etwa durch TGF-beta (transforming growth factor beta) oder Endothelin-1 vermittelte Entwicklung einer myokardialen Fibrose. Es besteht daher der Bedarf an alternativen Methoden, die eine Herzfibrose verringern oder vermeiden können.

Überraschenderweise wurde nun gefunden, dass eine Inhibierung des Proteasomsystems effektiv eine Herzfibrose verringern bzw. vermeiden kann, was zu einer verbesserten Herzfunktion führt.

Gegenstand der vorliegenden Erfindung ist daher die Verwendung mindestens eines Proteasom-Inhibitors zur Herstellung eines Medikamentes zur Behandlung fibrotischer Erkrankungen. Die fibrotischen Erkrankungen können unterschiedlichste Organsysteme, wie Lunge, Leber, Haut, Gelenke, Skelett und/oder Drüsen betreffen. Insbesondere bezieht sie sich auf fibrotische Erkrankungen des kardiovaskulären Systems.

Das Ubiquitin-Proteasom-System ist der Hauptstoffwechselweg für den Abbau von intrazellulären Proteinen in eukaryontischen Zellen, wie z.B. von Signalmediatoren, Zellzyklusproteinen und Transkriptionsfaktoren. Es konnte gezeigt werden, dass eine Inhibierung des Proteasoms die zelluläre Proliferation blockiert, mit verschiedenen Signalwegen interferiert und die Genexpression beeinflusst (Lee, D. H. et al. (1998) Trends Cell Biol., 8397-403; Yu C.-L. et al. (1997) J. Biol. Chem., 272, 14017-14020; Heldin C.-H. et al. (1999) Nat. Cell Biol., 1, E195-E197 und Desterro, J. M. P. et al., (2000) Cell Mol Live Sci., 57, 1207-1219).

Als Proteasom-Inhibitoren sind zum einen niedermolekulare organische Verbindungen und zum anderen molekularbiologische Verbindungen geeignet, die vorzugsweise im wesentlichen spezifisch das Proteasom inhibieren. Der Inhibitionstest kann beispielsweise wie bei Dahlmann B. et al. (2000) J. Mol. Biol., 303, 643-653 beschrieben durchgeführt werden. Der Begriff "im wesentlichen spezifisch" gemäß der vorliegenden Erfindung bedeutet, dass der Inhibitor das Proteasom stärker inhibiert als andere intrazelluläre Proteasen, wie z. B. Calpain, Cathepsine oder TPPII, vorzugsweise ca. 10mal stärker, insbesondere ca. 100mal stärker, vor allem ca. 1000mal stärker inhibiert als andere intrazelluläre Proteasen.

Die Inhibitionwerte werden üblicherweise als IC₅₀ (Konzentration des Inhibitors bei 50%iger Inhibition) angegeben und miteinander verglichen. Als niedermolekulare organische Verbindungen gemäß der vorliegenden Erfindung versteht man organische Verbindungen mit einer relativen Molmasse von kleiner gleich 1000, vorzugsweise kleiner gleich 800. Als molekularbiologische Verbindungen versteht man gemäß der vorliegenden Erfindung Nukleinsäuren, insbesondere RNA oder DNA, die die Expression einer Komponente des proteasomalen Systems inhibieren, beispielsweise die Transkription oder Translation der Proteasomkodierenden Nukleinsäuren, oder Proteine, insbesondere Bindepeptide oder -proteine, gegen mindestens eine Komponente des proteasomalen Systems, vorzugsweise gegen Ubiquitin und/oder gegen das Proteasom. Beispielsweise ist die Nukleinsäure eine anti-sense-RNA oder eine Doppelstrang-RNA (dsRNA) gegen eine Proteasom-kodierende Sequenz, ein Duplex-bildendes Oligonukleotid gegen eine Proteasom-kodierende Sequenz und/oder ein Knock-out-Konstrukt gegen eine Proteasom-kodierende Sequenz. Als Bindeproteine oder -peptide eignen sich beispielsweise Antikörper oder ihre bindeaktiven Teile, z. B. einzelkettige Antikörper (scAb) oder Fab-Fragmente, oder Derivate davon, z.B. bispezifische Antikörper, gegen mindestens eine Komponente des proteasomalen Systems. Eine Beschreibung des proteasomalen Systems und Proteasom-Inhibitoren findet sich beispielsweise in Kisselev A. F. & Goldberg A. L. (2001) Chemistry & Biology, 8, 739-758.

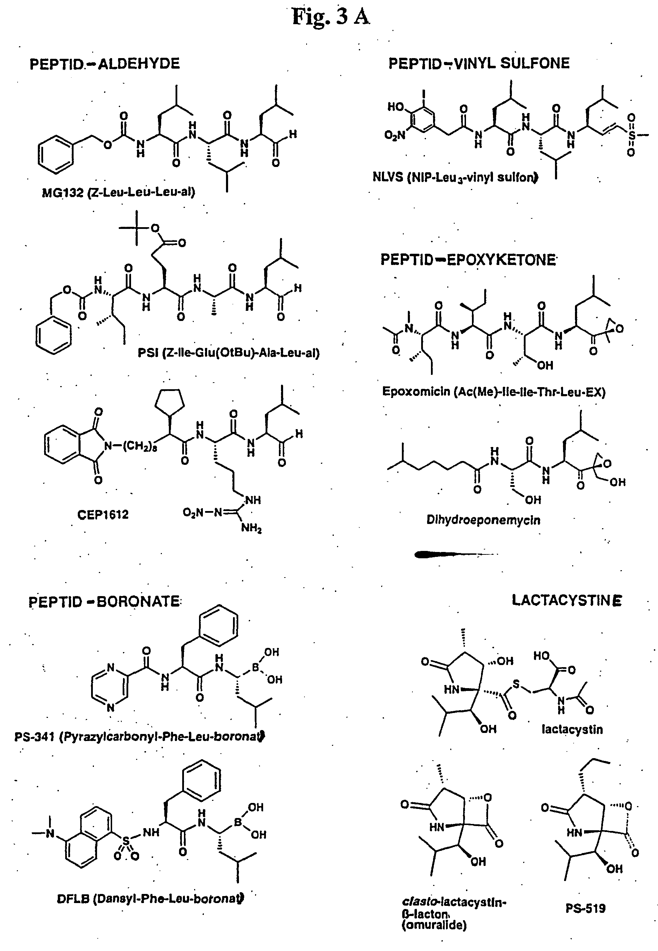

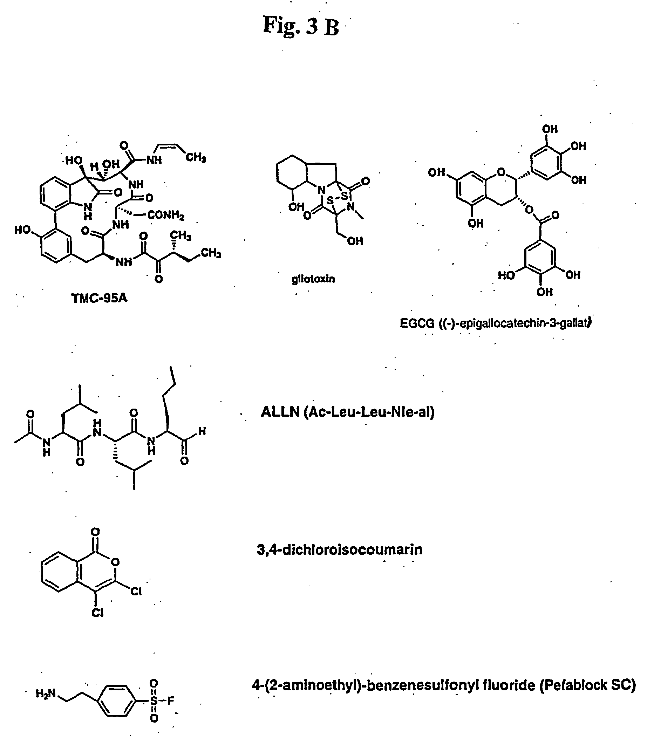

Demzufolge eignen sich als Proteasom-Inhibitoren gemäß Anspruch 1 insbesondere Threoninprotease-Inhibitoren, vor allem ein Peptid-Aldehyd, ein Peptid-Boronat, ein Peptid-Vinylsulfon, ein Peptid-Epoxyketon, ein Lactacystin, ein Peptid-alpha-Ketoaldehyd, ein alpha-Ketoamid, ein Indanonpeptid, ein Polyalkylenaldehyd und/oder ein Polyphenol, insbesondere ein Cathechin-3-gallat, welches z.B. aus grünem Tee gewonnen werden kann. Vor allem eignet sich der Proteasominhibitor Z-Leu-Leu-Leu-al (MG132), Z-Ile-Glu(OtBu)-Ala-Leu-al (PSI), CEP1612, Pyrazylcarbonyl-Phe-Leu-boronat (PS-341), Dansyl-Phe-Leu-boronat (DFLB), Morpholinonaphthylalanin-Leu-boronat (MG273), NIP-Leu₃-vinylsulfon (NLVS), Tyr-Leu₃-VS, NIP-Leu-Leu-Asn-VS, Ada-Tyr-Ahx₃-Leu₃-VS, Ada-Lys(Bio)-Ahx₃-Leu₃-VS Ac(Me)-Ile-Ile-Thr-Leu-EX (Epoxomicin), Dihydroeponemycin, Lactacystin, clasto-Lactacystin-beta-lacton (Omuralid), PS-519, Ac-Leu-Leu-Nle-al (ALLN), 3,4-Dichloroisocoumarin (DCI), 4-(2-Aminoethyl)-benzolsulfonylfluorid (Pefablock SC), TMC-95A, Gliotoxin, (-) Epigallocatechin-3-gallat (EGCG), Ritonavir, Lovastatin, Aclacinomicin A (Aclarubicin) und/oder Cyclosporin, die alle in Kisselev A. F. & Goldberg A. L. (2001, supra) und in Fig. 3 näher beschrieben sind, und worin Z eine Benzyloxycarbonylgruppe, al eine Aldehydgruppe, VS eine Vinylsulfongruppe, NIP eine 3-Nitro-4-hydroxy-5-iodophenylacetat-Gruppe und Bio eine Biotingruppe bedeutet. Besonders bevorzugt von allen Verbindungsklassen sind Threoninproteaseinhibitoren und davon insbesondere die Verbindungen MG132, MG262, Lactacystin und/oder Epoxomicin, vor allem MG132 und/oder MG262.

Gemäß der vorliegenden Erfindung wurden die Auswirkungen der Proteasom-Inhibierung auf die Herzfibrose in spontan hypertensiven Ratten (SH-Ratten) studiert. Überraschenderweise wurde bei einer Langzeitbehandlung über 12 Wochen mit dem spezifischen Proteasom-Inhibitor MG132 bei einer täglichen Dosis von 1 mg pro kg eine deutliche Inhibierung der Herzfibrose von ca. 40% festgestellt, wobei der Inhibitor sehr gut vertragen wurde. Die beschriebenen Effekte resultierten in einer verbesserten ventrikulären Funktion in den MG132-behandelten Tieren. In vitro wurde eine konzentrationsabhängige Inhibierung des Wachstums kardialer Fibroblasten und eine spezifische, konzentrationsabhängige Herabregulierung von Collagen Iα2 zu ca. 75% und von Collagen IIIα1 von ca. 90% beobachtet. Obwohl bei einer systemischen Behandlung mit Proteasom-Inhibitoren als Inhibitoren eines zentralen zellulären Systems Zytotoxizität zu erwarten gewesen wäre, wurden bei der beschriebenen Langzeitbehandlung keine messbaren Nebenwirkungen festgestellt, sondern im Gegenteil eine gute Verträglichkeit konstatiert. Somit eignen sich Proteasom-Inhibitoren in bevorzugter Weise zur Behandlung fibrotischer Erkrankungen insbesondere des kardiovaskulären Systems.

Die vorliegende Erfindung ist daher auch für die Behandlung von Patienten mit einer fibrotischen Erkrankung, vorzugsweise einer Fibrose des kardiovaskulären Systems verwendbar. Dabei ist sowohl eine systemische wie lokale Applikationsform möglich.

Die folgenden Methoden und Ergebnisse mit den Figuren 1 bis 3 sollen die Erfindung beispielhaft näher beschreiben, ohne sie darauf zu beschränken.

### Beschreibung der Figuren:

**Figur 1** beschreibt die Auswirkungen einer MG132 Behandlung von SH-Ratten auf die Herzfibrose bestimmt durch quantitative Morphometrie von Sirius-Rot-gefärbten links-ventrikulären mikroskopischen Schnitten (Abb. 1A-1C) und auf die Herzfunktion (Abb. 1D-1F).

**Figur 1A** zeigt einen repräsentativen Schnitt durch das Herz einer unbehandelten Kontroll-SH-Ratte, die eine gesteigerte myokardiale Fibrose zeigt. Das fibrotische Gewebe ist mit Sternchen beispielhaft markiert. Der Balken entspricht 20 µm.

**Figur 1B** zeigt einen typischen Schnitt durch das Herz eines MG132 behandelten Tieres, welches eine deutlich verringerte Herzfibrose unter Inhibition des Proteasoms zeigt.

**Figur 1C** zeigt die quantitative Auswertung mittels computerunterstützterBildanalyse. Dabei ergibt sich eine um ca. 40% verringerte Herzfibrose unter MG132-Behandlung. Mittelwert ± S.E.M. (standard error of mean = Standardabweichung/Wurzel aus n), n=7-10; *:p<0.05.

**Figur 1D** zeigt die enddiastolischen Drücke (LVEDP) im linken Ventrikel der Ratten mit deutlich verringertem Druckniveau bei den MG132-behandelten Tieren. Mittelwert ± S.E.M., n=6-10, *:p<0.05, **:p<0.01.

**Figur 1E** zeigt die maximale Druckanstiegsgeschwindigkeit (dp/dtmax), die bei den MG132-behandelten Tieren deutlich höher ist. Mittelwert ± S.E.M., n=6-10, *:p<0.05, **:p<0.01.

**Figur 1F** zeigt die maximale Druckabfallsgeschwindigkeit (dp/dtmin), die unter MG132 Behandlung um Faktor 2 höher ist. Mittelwert ± S.E.M., n=6-10, *:p<0.05, **:p<0.01.

**Figur 2** zeigt die Auswirkungen von Proteasom-Inhibitoren auf die Proliferation und Kollagenexpression in primären Herzfibroblasten der Ratte.

**Figur 2A** zeigt die dosisabhängige Inhibierung der Proliferation durch MG132. Die Zellen wurden mit 0,1 µM und 1 µM MG132 oder 0,1% DMSO stimuliert und täglich gezählt. Mittelwert ± S.E.M., n=3.

**Figur 2B** zeigt die Quantifizierung der mRNA-Menge der Kollagene Iα1, Iα2 und IIIα1 mittels "real-time" PCR-Analyse. Unter MG132-Behandlung kommt es dosisabhängig zu einer drastischen Senkung der Expression von Collagen Iα2 und IIIα1. Mittelwert ± S.E.M., n=2.

**Figur 3A** **und** **B** zeigen die chemische Struktur verschiedener Proteasom-Inhibitoren.

**Figur 4** zeigt eine real-time PCR-Analyse der Expression von MMP2 und MMP9 und der Kollagene Iα1, Iα2 und IIIα1 unter basalen Bedingungen (Fig. 4C bzw. Fig. 4D), sowie bei der Kostimulation mit MG132 und IL-β (Fig. 4A bzw. Fig. 4B).

**Figur 5** zeigt Zymographie-Experimente zum Nachweis von aktivem MMP2 und MMP9 unter basalen Bedingungen (Fig. 5A), bzw. bei der Kostimulation mit Proteasom-Inhibitor und IL-β (Fig. 5B).

**Figur 6** zeigt eine Bandshift-Analyse zum Nachweis von aktivem NFκB in Kernextrakten nach Stimulation mit MG132 und/oder mit IL-β.

### Methoden

### Methode 1: Gewinnung von Herzfibroblasten

Herzfibroblasten wurden aus neonatalen Wistar-Ratten durch Plattieren der Nicht-Myozyten Fraktion von neonatalen Herzen präpariert. Die Herzen wurden 2-3 Tage alten neonatalen Ratten entnommen. Ventrikuläres Gewebe wurde mit 50 mg pro ml Trypsin in Hanks-Balanced Salt Saline (HBSS) (Ca²⁺- und Mg²⁺-frei, Invitrogen™, Karlsruhe, Deutschland) über Nacht bei 4°C und mit Kollagenase (0,5 mg pro ml) in Leibowitzmedium (L-15, Invitrogen™, Karlsruhe, Deutschland) für 45 Minuten bei 37°C behandelt. Nach dem Waschen wurden die Zellen für eine Stunde bei 37°C vorplattiert. Die adhärenten Zellen waren hauptsächlich Herzfibroblasten, die unter Standardbedingungen in Standard-M199-Medium mit 10% Neugeborenenkalbserum, L-Glutamin und Penizillin-Streptomycin (Invitrogen™, Karlsruhe, Deutschland) weiterkultiviert wurden. In den Experimenten wurden subkultivierte Fibroblasten der Passagen 3-7 verwendet.

### Methode 2: Proliferationstest

MG132, ALLM und MG262 wurden von Calbiochem® (San Diego, Californien, USA) gekauft und als 10 mM DMSO-Stammlösungen bereitgestellt.

Herzfibroblasten (5x10⁴ Zellen pro ml) wurden auf 24 Lochplatten ausgesät. Adherente Zellen wurden entweder mit MG132 (0,1 und 1 µM) oder DMSO (0,1%) in Medium mit 10% Serum stimuliert und weiter für 7 Tage kultiviert. Das Medium wurde jeden zweiten Tag gewechselt. Die Proliferation wurde dadurch bestimmt, dass täglich die lebenden Zellen in dreifacher Ausfertigung mittels Trypan-Blau-Ausschlußmethode gezählt wurden. Diese Methode erlaubt die Unterscheidung zwischen lebenden und toten Zellen: Der Farbstoff Trypanblau, mit dem die Zellen versetzt werden, kann nur in Zellen mit einer defekten Zellmembran eindringen, die sich daraufhin blau färben. Lebende Zellen mit einer intakten Zellmembran werden nicht angefärbt.

### Methode 3: Histologische Bestimmung der Fibrose

Für die pathohistologische Evaluierung wurden die Rattenherzen in Paraffin eingebettet, in 3 µm dicke Schnitte zerteilt, einer Hämatoxylin-Eosin- und Sirius-Rot-Färbung unterworfen und wie in Hocher, B. et al., (1999), Hypertension, 3, 816-822 beschrieben, analysiert. Das Ausmaß der Herzfibrose wurde durch quantitative Morphometrie der Sirius-Rot-gefärbten Schnitte mit einem Bildanalysesystem (Quantimed 500, Image 1.61 Programm) evaluiert. Hierbei wurde das Verhältnis von rot gefärbtem Gewebe (Bindegewebe) gegen das Gesamtgewebe des Schnittes bestimmt.

### Methode 4: RNA und RT-PCR-Analyse

Gesamt-RNA wurde mittels Trizolreagenz (Invitrogen™, Karlsruhe, Deutschland) extrahiert. Anschließend wurde die RNA revers transkribiert (AMF Reverse Transkriptase, Roche Biochemicals, Mannheim, Deutschland) und mit DNase behandelt (Ambion, Huntingdon, UK). Es wurden PCR-Primer für die Rattenkollagen-RNAs Iα1, Iα2, IIIα1, für die RNAs der Matrix-Metalloproteinasen (MMP) 2 und 9 und für das "housekeeping" Gen Hypoxanthin-Phosphoribosyl-Transferase (HPRT) mit der Primerexpresssoftwareversion 1.2 (PerkinElmer/Applied Biosystems, Wellesley, MA, USA) entworfen und von TIB Molbiol, Berlin, Deutschland gekauft. Die PCR-Reaktion wurde gemäß den Empfehlungen des Herstellers mit SybrGreen in einem ABI PRISM 5700 Sequenzdetektor (PerkinEliner/Applied Biosystems, Wellesley, MA, USA) durchgeführt. Die relative Quantifizierung wurde mittels der vergleichenden C_{T}-Methode, wie vom Hersteller beschrieben, durchgeführt.

### Methode 5: Tierstudien

Altersgleiche männliche SH-Ratten wurden vom Jackson Laboratory, Maine, USA erhalten. Die Tiere wurden gemäß den internationalen Richtlinien für die Pflege und Verwendung von Labortieren gehalten. 6 Wochen alte Ratten wurden mit MG132 (1 mg pro kg Körpergewicht), 0,1% DMSO als Lösungsmittelkontrolle oder physiologischer Kochsalzlösung (n=10 pro Gruppe, tägliche intraperitoneale Injektionen) für 12 Wochen behandelt. Die Tiere wurden mit einer Hochsalzdiät gefüttert (Trinkwasser mit 2% Natriumchlorid). Der Blutdruck wurde wöchentlich mit Hilfe der Standard-Schwanzmanschetten-Methode gemessen. Die Blutwerte und Standardlabormarker der renalen und hepatischen Funktionen wurden 6 Wochen nach der Behandlung und am Ende der Studie durch Standardmethoden bestimmt. Am Ende der Studie wurden die Tiere mittels einer intraperitonealen Injektion einer 20%igen Urethanlösung (0,9 g pro kg Körpergewicht) anästhesiert und die links-ventrikulären Druckparameter wie bei Saragoca, M. et al. (1981) Hypertension, 3, 380-385 beschrieben, bestimmt. Die Organe wurden entfernt, gewogen und entweder in flüssigem Stickstoff schockgefroren oder in Paraffin für die histologische Analyse eingebettet. Da keine Differenz zwischen DMSO- und salzbehandelten SH-Ratten bezüglich der oben genannten Parameter beobachtet wurde, dienten die DMSO-behandelten Tiere als Kontrolle für die MG132-Behandlung.

Die Daten wurden als Mittelwerte ± S.E.M. bestimmt, sofern nichts anderes vermerkt wurde. Die Signifikanz der Differenz in den links-ventrikulären Druckparametern und in der Quantifizierung der Herzfibrose wurden nach dem Student's T-Test bestimmt. Für den Zellproliferationstest wurde die Signifikanz durch Vergleich der Regressionskoeffizienten von MG132 gegen die Kontrollgruppe gemessen. Eine Fehlerwahrscheinlichkeit von p<0,05 wurde als signifikant erachtet. Für alle statistischen Kalkulationen wurde die SPSS 9.0 Software benutzt.

### Ergebnisse

Die Behandlung von spontan hypertensiven Ratten mit dem spezifischen Proteasom-Inhibitor MG132 über 12 Wochen wurde im Allgemeinen über diese langen Zeitraum gut vertragen. In nach 6 Wochen und am Ende der Studie genommenen Blutproben wurden keine Veränderungen im Differentialblutbild oder eine Veränderung der Labormarker, die Nebenwirkungen der Niere oder Leber zeigen, beobachtet. Darüber hinaus zeigten die MG132-behandelten Tiere keine signifikante Änderung im systolischen Blutdruck und im Herzgewicht (Tabelle 1).

**Tabelle 1**

| | **Kontrolle** | **1 mg/kg MG132** |
|---|---|---|
| Systolischer BD (mm Hg) | 196,75±9,1 | 191,43±11,2 |
| Körpergewicht (g) | 286±36,6 | 280±15,3 |
| Herzgewicht (g) | 1,28±0,07 | 1,18±0,07 |
| HG/KG(mg/g) | 4,64±0,61 | 4,1±0,34 |

### BD steht für Blutdruck; KG für Körpergewicht und HG für Herzgewicht

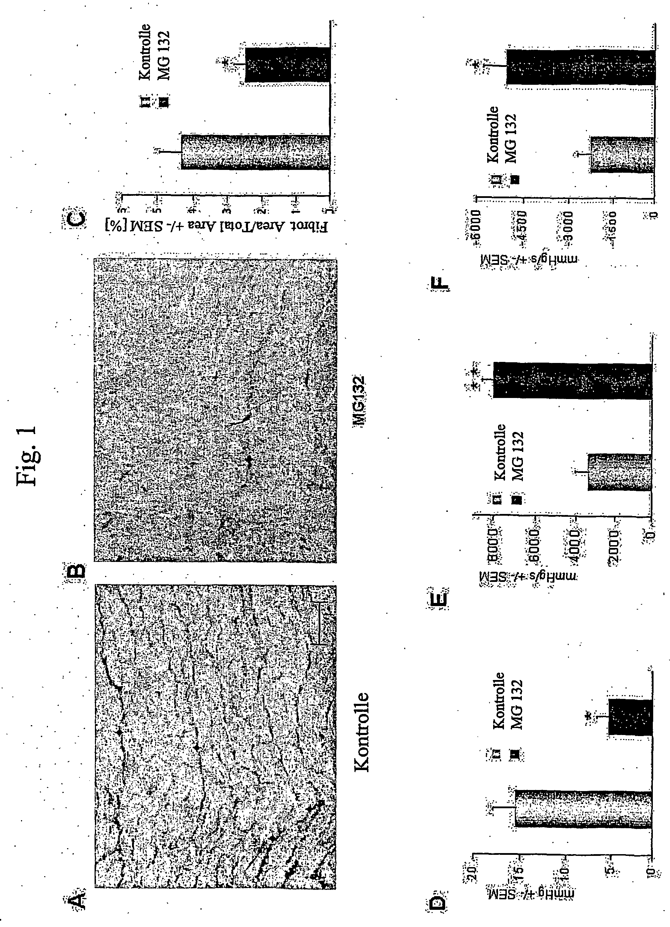

Wie aus den Figuren 1A bis 1C ersichtlich ist, zeigten MG132 behandelte SH-Ratten eine deutliche Reduktion (-38%) der Herzfibrose verglichen mit den Kontrolltieren bestimmt durch quantitative Morphometrie von Sirius-Rot gefärbten Sektionen der linken Ventrikel.

Die Prävention der Herzfibrose in MG132-behandelten SH-Ratten korrelierte gut mit der normalen links-ventrikulären Funktion, wobei die Kontrollen Anzeichen einer beginnenden links-ventrikulären Dysfunktion zeigten, wie in den Figuren 1D-1F dargestellt: Die Füllungsdrücke (LVEDP) waren deutlich niedriger, (Figur 1D: 15±2 versus 5±3 mm Hg, p=0,017), die maximale Druckanstiegsgeschwindigkeit dp/dtmax (Figur 1E: 8010±538 versus 3375±662 mm Hg, p=0,003) und die maximale Druckabfallsgeschwindigkeit dp/dtmin (Abb. 1F:-5046±726 versus -2290±422 mm Hg/s, p=0,015) - die Parameter der Herzinotropie und Herzlusitropie sind - waren mehr als doppelt so hoch in den MG132-behandelten SH-Ratten verglichen zu den Kontroll-SH-Ratten.

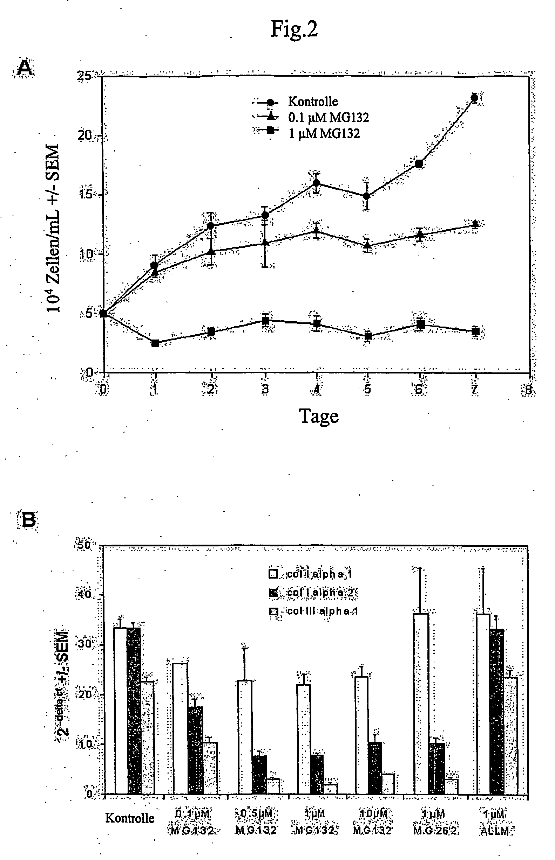

Um mögliche Mechanismen aufzuklären, wie die Proteasom-lnhibierung zu der verringerten Herzfibrose *in vivo* beitragen kann, wurden primäre Herzfibroblasten der Ratte mit Proteasom-Inhibitoren behandelt. Wie in Figur 2A dargestellt, verursachte die Behandlung von Herzfibroblasten mit 0,1 und 1 µM MG132 eine Dosis-abhängige Inhibierung der Proliferation. Darüber hinaus wurde die RNA-Expression von Collagen Iα2 und IIIα1 in dosisabhängiger Weise durch MG132 bis zu -73 % und bis zu 91 % inhibiert (Figur 2B). Im Gegensatz dazu war die Collagen Iα1 Expression unbeeinflusst. Ein zweiter spezifischer Proteasom-Inhibitor MG262, welcher ein Boronatderivat von MG132 ist, inhibierte die Kollagenexpression ähnlich effektiv (Figur 2B) und belegt somit die Spezifität der proteasomalen Inhibition. Der Cathepsininhibitor ALLM, der als Peptid-Aldehyd (A-LLM-al) strukturell verwandt mit MG132 ist, zeigte demgegenüber keine Effekte auf die Kollagenexpression (Figur 2B).

Die endemisch auftretenden und gesundheitspolitisch extrem wichtigen Organfibrosen (besonders Herzmuskelfibrose) sind von extremen Varianten von Fibrosebildungen in Form von Entzündungsreaktionen auf Fremdkörper völlig zu trennen (vergleiche auch Kapselbildung bei Silikonimplantaten), über die H. Rupp, P. Newrzella, H. König und B. Maisch in: "Hemmung der kardialen Fibrose durch Blockierung des Transkriptionsfaktors NF-kappaB" auf der 67. Jahrestagung der Deutschen Gesellschaft für Kardiologie - Herz- und Kreislaufforschung, 19.-21. April 2001, Mannheim, berichteten.

Speziell bei der millionenfach vorkommenden, erfindungsgemäß durch die Verabreichung von Proteasom-Inhibitoren zur Prävention oder Therapie behandelbare Herzmuskelfibrosierung ist das pathophysiologische Agens die neuroendokrine Aktivierung mit der Freisetzung vasokonstriktorischer Mediatoren wie Katecholamine, Angiotensin II, Endothelin-1, TGF-beta und anderen, die im Rahmen einer globalen Überlast (z. B. chronische Druckbelastung bei arterieller Hypertension) oder einer regionalen Überlast (z. B. kompensatorische Hyperkinesie des intakten Restmyokards bei Myokardinfarkt) verstärkt liberiert werden. Neben ihren vasokonstriktorischen Eigenschaften haben diese Mediatoren massive wachstumsinduzierende Effekte im Sinne der Massenzunahme von Herzmuskelzellen und der Proliferation und vermehrten Syntheseleistung in Form von Extrazellulärmatrixbildung von kardialen Fibroblasten.

Bei den erfindungsgemäß mit Proteasom-Inhibitoren behandelten, durch Überlastungen geschädigten fibrotischen Herzen werden in der feingeweblichen Untersuchung in der Regel keine Hinweise auf eine inflammatorische Reaktion beobachtet. Es ist wissenschaftlich gut belegt, dass diese durch Überlast induzierten kardialen Fibrosen unabhängig von der Aktivierung des inflammatorischen Transkriptionsfaktors NFκB sind und über die Signalkette des TGF-beta laufen (Siehe Kitamoto et al., Circulation 2000;102:806-12; Lijnen et al., Mol Gen Metab 2000;71:418-35). Die erfindungsgemäß durch Proteasom-Inhibitoren behandelbare kardiovaskulär relevante Fibrosierung wird folglich unabhängig von NFκB durch verschiedene Formen der Überlast mit konsekutiver neuroendokriner Aktivierung verursacht.

Diese Aussagen werden eindeutig dadurch gestützt, dass die wesentlichen Substanzgruppen, die gegen die Entwicklung einer Fibrose gerichtet sind, oder die zur Regression einer Fibrose beitragen, antagonistische Wirkungen auf spezifische vasoaktive Mediatoren haben. Gute Beispiele dafür sind Angiotensin Converting Enzyme-Inhibitoren (ACE-Hemmer), Angiotensin II Typ 1-Rezeptor Antagonisten (AT-1-Antagonisten) und Endothelin-Rezeptorantagonisten.

Andere bevorzugte Ausführungsbeispiele von erfindungsgemäß mittels Proteasom-Inhibitoren behandelbaren Organfibrosen ohne dominierende inflammatorische Komponente sind stauungsbedingte Fibrosierung der Leber, Hochdruckbedingte Fibrosierung der Nieren, Gelenkfibrosen bei Fehlstellungen.

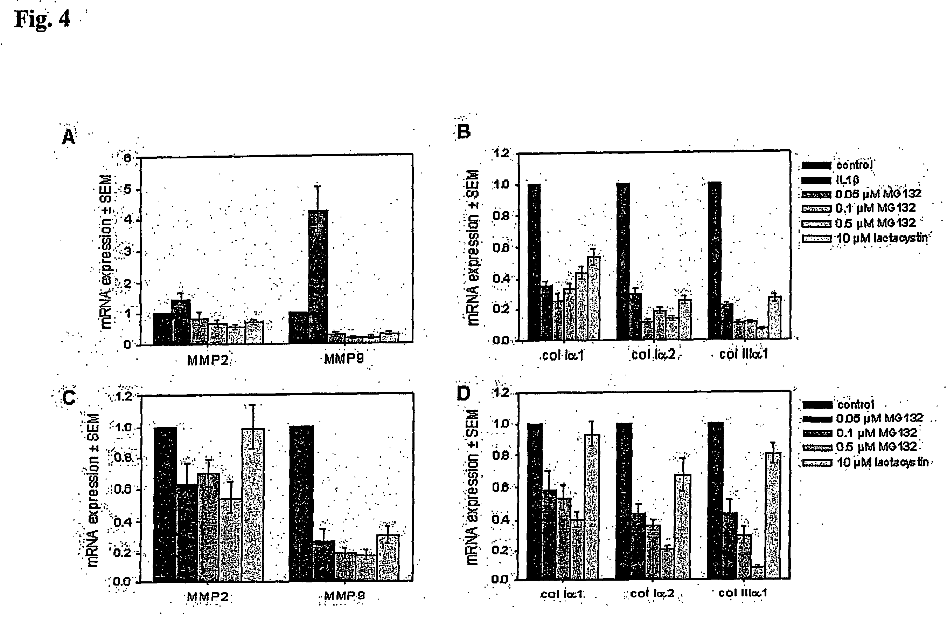

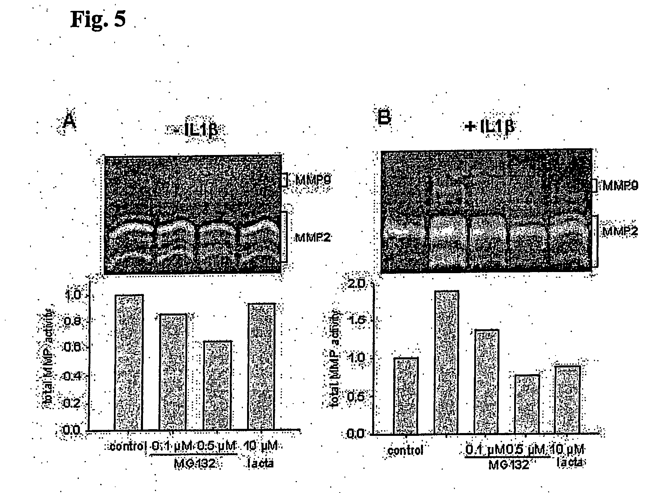

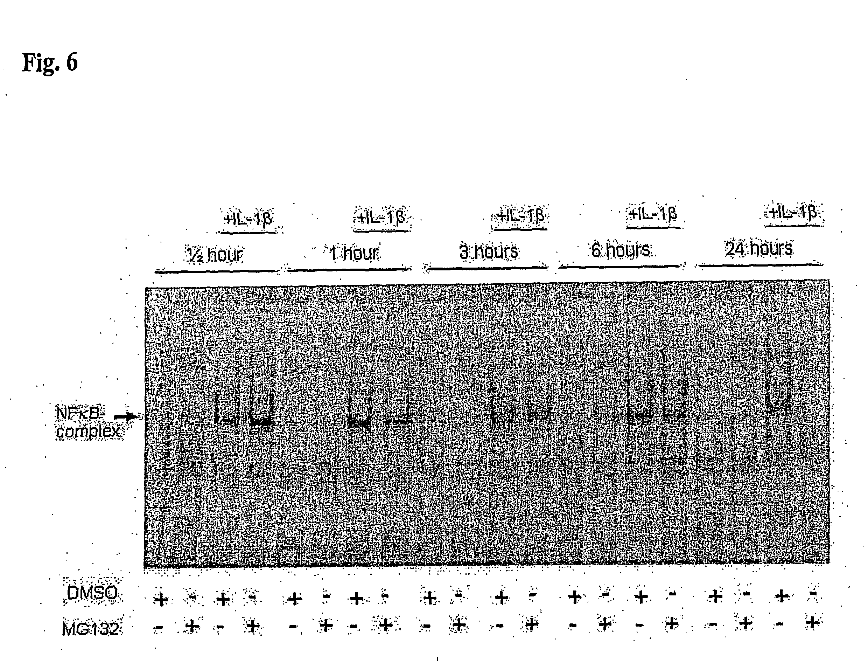

Experimentelle Daten der vorliegenden Erfindung belegen, dass die erfindungsgemäßen anti-fibrotischen Effekte von Proteasom-Inhibitoren unabhängig von der Aktivierung des inflammatorischen Transkriptionsfaktors NFκB sind.
1. Real-time PCR-Analyse der Expression von Kollagenen und MMP2 und 9 (Fig. 4):
   Hierzu wurden kardiale Fibroblasten unter serum-freien Bedingungen 24 Stunden mit Proteasom-Inhibitoren behandelt und aus der präparierten RNA die Expression der Kollagene Iα1, Iα2 und IIIα1 und von MMP2 und 9 mittels real-time RT-PCR bestimmt. Wie in Fig. 4C und 4D gezeigt, wurde durch die erfindungsgemäße Inhibition des Proteasoms nicht nur die Expression der Kollagene sondern auch die der MMPs deutlich inhibiert. Diese inhibitorische Wirkung der Proteasom-Inhibitoren war erfindungsgemäß unter basalen Bedingungen und ohne zusätzliche Zytokinstimulation der Zellen zu beobachten.
   Um zu untersuchen, ob Proteasom-Inhibitoren auch nach Induktion der MMPs durch Stimulation mit dem NFκB-aktivierenden Zytokin Interleukin-1β (IL-1β) diese Effekte zeigen, wurden die kardialen Fibroblasten mit IL-1β und Proteasom-Inhibitor kostimuliert.
   Die IL-1β-Stimulation führte zu einer deutlichen Expressionssteigerung der MMPs, insbesondere von MMP9 (Fig. 4A). Diese MMP ist als eine durch NFκB aktivierbare und durch Zytokinbehandlung stimulierbare Protease beschrieben (Gum et al., J Biol Chem. 1996; 271:10672-80). Die gleichzeitige Gabe von Proteasom-Inhibitoren verhinderte nicht nur den IL-1β-induzierten Anstieg der Expression, sondern reduzierte überraschender Weise die Expression bis unter die DMSO-Kontrollwerte (Fig. 4A). Dies belegt die erfindungsgemäße Hemmung der Expression der MMPs durch Proteasom-Inhibierung, die zu einer stärkeren Hemmung der MMP-Expression führt als zu einer Hemmung der von IL-1β vermittelten MMP-Expression und somit unabhängig von der Hemmung der Aktivierung von NFκB ist.
   Noch deutlicher konnte die erfindungsgemäße, von einer Inhibierung von NFκB unabhängige Wirkung von Proteasom-Inhibitoren bei der erfindungsgemäßen Prävention und Behandlung von fibrotischen Erkrankungen in einer Analyse der Kollagenexpression unter IL-1β Stimulation gezeigt werden (Fig. 4B).
   Wie bereits von Siwik et al., (Siwik et al., Am J Physiol Cell Physiol. 2001;280:C53-C60) beschrieben, führte die IL-1β Behandlung zu einer Herabregulation der Kollagenexpression in kardialen Fibroblasten (Fig. 4B). Diese Suppression lässt sich durch negativ regulatorische NFκB-Elemente im Promotor der Kollagene, d.h. durch eine suppressorische Wirkung von aktiviertem NFκB auf die Expression der Kollagene, erklären (Kouba et al., J Immunol. 1999;162:3226-34). Die Kostimulation der Fibroblasten mit IL-1β und mit Proteasom-Inhibitoren resultierte jedoch nicht in einer Aufhebung dieser Suppression der Kollagene durch NFκB, wie bei der Inhibierung der Aktivierung von NFκB durch Proteasomeninhibitoren zu erwarten gewesen wäre, sondern führte überraschender Weise zu einer noch stärkeren Suppression der Kollagenexpression als mit IL-1β alleine (Fig. 4B).
   Die vorliegenden Untersuchungen belegen, dass die erfindungsgemäße Behandlung von fibrotischen Erkrankungen mittels Proteasominhibitoren unabhängig von der Inhibierung der Aktivierung von NFκB ist. Vorteilhafter Weise ermöglicht die Erfindung die Inhibierung der Expression von MMPs und Kollagenen bei fibrotischen Erkrankungen unabhängig von der Inhibierung der Aktivierung von NFκB. Vorzugsweise ermöglicht die Erfindung die Prävention und Behandlung von fibrotischen Erkrankungen, die nicht oder nicht vorrangig von NFκB vermittelt werden.
2. Zymographie zum Nachweis von aktivem MMP2 und MMP9 (Fig. 5):
   Die erfindungsgemäße Inhibierung der Expression aktiver MMP2 in kardialen Fibroblasten unter basalen, d.h. nicht durch NFκB stimulierten, Bedingungen mittels Proteasom-Inhibitoren wurde auch in Zymographie-Experimenten bestätigt (Fig. 5). Hierzu wurden die Zellkulturüberstände nach Inkubation mit Proteasom-Inhibitoren in Abwesenheit (Fig. 5A) bzw. Anwesenheit (Fig. 5B) von Il-1β bezüglich ihrer Gelatinase-Aktivität mittels Zymographie untersucht.
   Erfindungsgemäß wurde die MMP2-Bildung durch den Proteasom-Inhibitor bereits in Abwesenheit von Il-1β, d.h. unabhängig von NFκB, verringert (Fig. 5A). Die durch Il-1β induzierte MMP2- und MMP9-Aktivierung wurde durch gleichzeitige Gabe von Proteasom-Inhibitoren ebenfalls verringert (Fig. 5B).
3. Bandshift-Analyse zum Nachweis aktiven NFκBs (Fig. 6):
   Die oben dargestellten Ergebnisse belegen, dass die erfindungsgemäßen inhibitorischen Wirkungen der Proteasom-Inhibitoren auf die Kollagen- und MMP-Expression bei unstimulierten kardialen Fibroblasten auftreten und deshalb unabhängig von der Inhibierung der Aktivierung von NFκBsind. Dies wurde zusätzlich in Bandshift-Analysen mit Oligonukleotiden, die eine NFκB-DNA-Bindungstelle aufweisen, bestätigt. Dazu wurden Kernextrakte von kardialen Fibroblasten hergestellt, die in einer Zeitreihe in An- und Abwesenheit von Il-1β mit 0.5 µM MG132 bzw. DMSO (Lösungsmittelkontrolle) behandelt wurden.
   Wie in Fig. 6 gezeigt, weisen kardiale Fibroblasten unter nicht-stimulierten, basalen Bedingungen keinen aktiven NFκB-Komplex auf (siehe Kontrollbanden). Unter Il-1 β-Stimulation kommt es zu einer Aktivierung von NFκB, dargestellt als Bandshift. Die zeitgleiche Behandlung mit 0.5 µM MG132 zeigte erst nach 6 Stunden einen leichten inhibitorischen Effekt auf NFκB, der die überraschend starke Verringerung der Expression von MMP2 und MMP9 bei der gleichzeitigen Gabe von MG132 und IL-1β (s.o. Fig. 4A) nicht zu erklären vermag. Nach 24 Stunden war eine Verringerung der durch I1-1β-induzierten Aktivierung von NFκB mit und ohne MG132 zu beobachten.

Die in vitro Experimente belegen somit, dass die erfindungsgemäßen inhibitorischen Effekte von Proteasom-Inhibitoren auf die Expression von Kollagenen und MMPs unabhängig vom inflammatorischen Transkriptionsfaktor NFκB vermittelt werden.

Auch die eingangs dargestellten Daten zur erfindungsgemäßen Verhinderung der kardialen Fibrose im Tiermodell der spontan hypertensiven Ratten belegen die erfindungsgemäße, von Entzündungsreizen unabhängige Suppression der Kollagenexpression durch MG132: Dieses Tiermodell geht erfindungsgemäß nicht von einem Entzündungsreiz als Auslöser für die kardiale Fibrose aus, sondern von einer ausgeprägten Hypertonie der Ratten.

Erfindungsgemäß greifen Proteasom-Inhibitoren nicht nur inhibierend in die Kollagen- und MMP-Expression ein, sondern zeigen ihr breites Wirkspektrum auch in der Suppression der Fibroblastenproliferation.

Die erfindungsgemäßen therapeutischen Interventionen umfassen vorzugsweise die systemische Gabe von Proteasom-Inhibitoren. Vorzugsweise wird dabei einem Patienten wenigstens ein Proteasom-Inhibitor in Dosis verabreicht, in der vorteilhafter Weise systemische Nebenwirkungen, insbesondere zytotoxische Nebenwirkungen, von Proteasom-Inhibitoren nicht oder nur in geringem Maße auftreten. Die erfindungsgemäße Verwendung von Proteasom-Inhibitoren zur Prävention und Therapie von Fibrosen ist deshalb gut verträglich, ermöglicht vorzugsweise eine spezifische anti-fibrotische Behandlung und ermöglicht somit eine erfindungsgemäße Prävention und Therapie bei einer Vielzahl von Patienten.

Bei bevorzugten Ausführungen ermöglicht eine erfindungsgemäße systemische Gabe wenigstens eines Proteasom-Inhibitors eine Prävention und Behandlung vorzugsweise einer kardialen Fibrose, vorzugsweise einer durch Überlast verursachten kardialen Fibrose, vorzugsweise einer durch Überlast bei chronischer Druckbelastung bei arterieller Hypertension verursachten kardialen Fibrose, vorzugsweise einer durch Überlast bei kompensatorischer Hyperkinesie des intakten Restmyokards bei Myokardinfarkt verursachten kardialen Fibrose, vorzugsweise einer durch Überlast mit konsekutiver neuroendokriner Aktivierung verursachten kardialen Fibrose, vorzugsweise einer kardialen Fibrose, bei der eine Behandlung durch ACE-Hemmer, AT-1-Antagonisten und/oder Endothelin-Rezeptorantagonisten indiziert ist, vorzugsweise einer stauungsbedingten Fibrosierung der Leber, vorzugsweise einer Hochdruck-bedingten Fibrosierung der Nieren und vorzugsweise aller Gelenkfibrosen bei Fehlstellungen von Gelenken.

Bei der erfindungsgemäßen Prävention und der erfindungegemäßen Therapie von Fibrosen wird einem Patienten wenigstens ein Proteasom-Inhibitor, vorzugsweise in einer Dosierung von in etwa 0,5 µg/kg Körpergewicht bis in etwa 0,5 mg/kg Körpergewicht, vorzugsweise in einer Dosierung von in etwa 1 µg/kg Körpergewicht bis in etwa 0,1 mg/kg Körpergewicht, vorzugsweise in einer Dosierung von in etwa 0,01 mg/kg Körpergewicht bis in etwa 0,1 mg/kg Körpergewicht verabreicht. Diese Dosierungen beziehen sich auf alle hierin genannten Proteasom-Inhibitoren, insbesondere die Threoninprotease-Inhibitoren, insbesondere MG132 und MG262. Vorzugsweise wird wenigsten einer der hierin eingangs erwähnten Proteasom-Inhibitoren verabreicht, vorzugsweise MG132. Dabei werden vorzugsweise vorteilhafte Verabreichungsformen verwendet, die dem Fachmann bekannt sind, vorzugsweise feste und flüssige Arzneimittelzubereitungen, vorzugsweise Infusionslösungen, die vorzugsweise wenigstens einen vorteilhaften Arzneimittelzusatz enthalten, der dem Fachmann geläufig ist und der zur Verbesserung der Lagerfähigkeit und/oder der Verträglichkeit der Arzneimittelzubereitung beiträgt.

## Patentansprüche

1. Verwendung mindestens eines Proteasom-Inhibitors zur Herstellung eines Medikaments zur Behandlung fibrotischer Erkrankungen, die nicht durch Entzündungsreaktionen auf Fremdkörper verursacht sind,
**dadurch gekennzeichnet, dass** der Proteasom-Inhibitor ein Threoninprotease-Inhibitor, ein Genexpressionsinhibitor des proteasomalen Systems und/oder ein Bindeprotein oder -peptid gegen mindestens eine Komponente des proteasomalen Systems, vorzugsweise gegen Ubiquitin und/oder gegen das Proteasom ist.

2. Verwendung nach Anspruch 1 zur Herstellung eines Medikaments zur Behandlung einer durch Überlast verursachten kardialen Fibrose, einer stauungsbedingten Fibrosierung der Leber, einer Hochdruck-bedingten Fibrosierung der Nieren oder einer Gelenkfibrose bei Fehlstellung eines Gelenks.

3. Verwendung nach Anspruch 2 zur Herstellung eines Medikaments zur Behandlung einer durch Überlast bei chronischer Druckbelastung bei arterieller Hypertension verursachten kardialen Fibrose und/oder zur Behandlung einer durch Überlast bei kompensatorischer Hyperkinesie des intakten Restmyokards bei Myokardinfarkt verursachten kardialen Fibrose.

4. Verwendung nach Anspruch 2 oder 3 zur Herstellung eines Medikaments zur Behandlung einer kardialen Fibrose, bei der eine Behandlung durch ACE-Hemmer, AT-1-Antagonisten und/oder Endothelin-Rezeptorantagonisten indiziert ist.

5. Verwendung nach einem oder mehreren der Ansprüche 1 bis 4, wobei einem Patienten wenigstens ein Proteasom-Inhibitor in einer Dosierung von in etwa 0,5 µg/kg Körpergewicht bis in etwa 0,5 mg/kg Körpergewicht, vorzugsweise von in etwa 1 µg/kg Körpergewicht bis in etwa 0,1 mg/kg Körpergewicht, vorzugsweise von in etwa 0,01 mg/kg Körpergewicht bis in etwa 0, 1 mg/kg Körpergewicht verabreicht wird.

6. Verwendung nach einem oder mehreren der Ansprüche 1 bis 5, **dadurch gekennzeichnet, dass** die fibrotischen Erkrankungen fibrotische Erkrankungen von Organen, vorzugsweise von Lunge, Leber, Haut, Gelenke, Skelett und/oder Drüsen betreffen, insbesondere Erkrankungen des kardiovaskulären Systems.

7. Verwendung nach einem oder mehreren der Ansprüche 1 bis 6, **dadurch gekennzeichnet, dass** der Proteasom-Inhibitor eine niedermolekulare organische Verbindung oder eine molekularbiologische Verbindung ist.

8. Verwendung nach Anspruch 7, **dadurch gekennzeichnet, dass** der Proteasom-Inhibitor ein Peptid-Aldehyd, ein Peptid-Boronat, ein Peptid-Vinylsulfon, ein Peptid-Epoxyketon, ein Lactacystin, ein Peptid-alpha-Ketoaldehyd, ein alpha-Ketoamid, ein Indanonpeptid, ein Polyalkylenal-dehyd, ein Polyphenol, insbesondere ein Cathechin-3-gallat, eine Nuklein-säure gegen mindestens eine Komponente des proteasomalen System und/oder ein Antikörper oder ihre bindeaktiven Teile oder Derivate davon gegen mindestens eine Komponente des proteasomalen Systems ist.

9. Verwendung nach einem der Ansprüche 7 oder 8, **dadurch gekennzeichnet, dass** der Proteasominhibitor Z-Leu-Leu-Leu-al (MG132), Z-Ile-Glu(OtBu)-Ala-Leu-al (PSI), CEP1612, Pyrazylcarbonyl-Phe-Leu-boronat (PS-341), Dansyl-Phe-Leu-boronat (DFLB), Morpholinonaphthylalanin-Leu-boronat (MG273), NIP-Leu₃-vinylsulfon (NLVS), Tyr-Leu₃-VS, NIP-Leu-Leu-Asn-VS, Ada-Tyr-Ahx₃-Leu₃-VS, Ada-Lys(Bio)-Ahx₃-Leu₃-VS Ac(Me)-Ile-Ile-Thr-Leu-EX (Epoxomicin), Dihydroeponemycin, Lactacystin, clasto-Lactacystin-beta-lacton (Omuralid), PS-519, Ae-Leu-Leu-Nle-al (ALLN), 3,4-Dichloroisocoumarin (DCI), 4-(2-Aminoethyl)-benzolsulfonylfluorid (Pefablock SC), TMC-95A, Gliotoxin, (-)Epigallocatechin-3-galllat (EGCG), Ritonavir, Lovastatin, Aclacinomicin A (Aclarubicin), Cyclosporin, anti-sense-RNA oder Doppelstrang-RNA (dsRNA) gegen eine Proteasom-kodierende Sequenz, Triplex-bildendes Oligonukleotid gegen eine Proteasom-kodierende Sequenz und/oder ein Knock-out-Konstrukt gegen eine Proteasom-kodierende Sequenz ist, worin Z eine Benzyloxycarbonylgruppe, al eine Aldehydgruppe, VS eine Vinylsulfongruppe, NIP eine 3-Nitro-4-hydroxy-5-iodophenylacetat-Gruppe und Bio eine Biotingruppe bedeutet.

## Claims

1. Use of at least one proteasome inhibitor to prepare a medicament for the treatment of fibrotic diseases which are not caused by inflammatory reactions to foreign bodies, **characterized in that** the proteasome inhibitor is a threonine protease inhibitor, a gene expression inhibitor of the proteasomal system and/or a binding protein or binding peptide directed against at least one component of the proteasomal system, preferably against ubiquitin and/or against the proteasome.

2. Use according to claim 1 to prepare a medicament for the treatment of a cardiac fibrosis caused by overload, a liver fibrosis caused by congestion, a kidney fibrosis caused by high pressure or a joint fibrosis in the case of a joint malposition.

3. Use according to claim 2 to prepare a medicament for the treatment of a cardiac fibrosis caused by overload under chronic pressure stress in the case of arterial hypertension and/or for the treatment of a cardiac fibrosis caused by overload in compensatory hyperkinesia of the intact residual myocardium in the case of myocardial infarction.

4. Use according to claim 2 or 3 to prepare a medicament for the treatment of a cardiac fibrosis in which a treatment with ACE inhibitors, AT-1 antagonists and/or endothelin receptor antagonists is indicated.

5. Use according to one or more of claims 1 to 4, wherein a patient is administered at least one proteasome inhibitor in a dose from approximately 0.5 µg/kg of body weight to approximately 0.5 mg/kg of body weight, preferably from approximately 1 µg/kg of body weight to approximately 0.1 mg/kg of body weight, preferably from approximately 0.01 mg/kg of body weight to approximately 0.1 mg/kg of body weight.

6. Use according to one or more of claims 1 to 5, **characterized in that** the fibrotic diseases are fibrotic diseases of organs, preferably of the lung, liver, skin, joints, skeleton and/or glands, in particular diseases of the cardiovascular system.

7. Use according to one or more of claims 1 to 6, **characterized in that** the proteasome inhibitor is a low-molecular organic compound or a molecular-biological compound.

8. Use according to claim 7, **characterized in that** the proteasome inhibitor is a peptide aldehyde, a peptide boronate, a peptide vinyl sulphone, a peptide epoxyketone, a lactacystin, a peptide alpha-ketoaldehyde, an alpha-ketoamide, an indanone peptide, a polyalkylene aldehyde, a polyphenol, in particular a cathechin-3-gallate, a nucleic acid directed against at least one component of the proteasomal system and/or an antibody or binding-active parts or derivatives thereof directed against at least one component of the proteasomal system.

9. Use according to one of claims 7 or 8, **characterized in that** the proteasome inhibitor is Z-Leu-Leu-Leu-al (MG132), Z-Ile-Glu(OtBu)-Ala-Leu-al (PSI), CEP1612, pyrazylcarbonyl-Phe-Leu-boronate (PS-341), dansyl-Phe-Leu-boronate (DFLB), morpholino-naphthylalanine-Leu-boronate (MG273), NIP-Leu₃-vinylsulphone (NLVS), Tyr-Leu₃-VS, NIP-Leu-Leu-Asn-VS, Ada-Tyr-Ahx₃-Leu₃-VS, Ada-Lys(Bio)-Ahx₃-Leu₃-VS, Ac(Me)-Ile-Ile-Thr-Leu-EX (epoxomicin), dihydroeponemycin, lactacystin, clasto-lactacystin-beta-lactone (omuralide), PS-519, Ac-Leu-Leu-Nle-al (ALLN), 3,4-dichloroisocoumarin (DCI), 4-(2-aminoethyl)-benzenesulphonyl fluoride (Pefablock SC), TMC-95A, gliotoxin, (-)epigallocatechin-3-gallate (EGCG), ritonavir, lovastatin, aclacinomycin A (aclarubicin), cyclosporin, an antisense RNA or a double-stranded RNA (dsRNA) directed against a proteasome-encoding sequence, a triplex-forming oligonucleotide directed against a proteasome-encoding sequence and/or a knockout construct directed against a proteasome-encoding sequence, where Z is a benzyloxycarbonyl group, al is an aldehyde group, VS is a vinyl sulphone group, NIP is a 3-nitro-4-hydroxy-5-iodophenylacetate group, and Bio is a biotin group.

## Revendications

1. Utilisation d'au moins un inhibiteur de protéasome pour la fabrication d'un médicament destiné au traitement de maladies fibrotiques, qui ne sont pas provoquées par des réactions inflammatoires à corps étrangers,
**caractérisée en ce que** l'inhibiteur de protéasome est un inhibiteur de thréonine protéase, un inhibiteur d'expression génique du système protéasomique et/ou une protéine ou un peptide de liaison contre au moins un composant du système protéasomique, de préférence contre une ubiquitine et/ou contre le protéasome.

2. Utilisation selon la revendication 1 pour la fabrication d'un médicament destiné au traitement d'une fibrose cardiaque provoquée par une surcharge, d'une fibrose hépatique congestive, d'une fibrose rénale provoquée par une hypertension ou d'une fibrose articulaire en cas de malposition d'une articulation.

3. Utilisation selon la revendication 2 pour la fabrication d'un médicament destiné au traitement d'une surcharge en cas de charge de tension chronique dans une fibrose cardiaque provoquée par une hypertension artérielle et/ou pour le traitement d'une hyperkinésie compensatoire du myocarde encore intact dans le cas d'une surcharge dans une fibrose cardiaque provoquée par un infarctus du myocarde.

4. Utilisation selon la revendication 2 ou 3 pour la fabrication d'un médicament destiné au traitement d'une fibrose cardiaque, dans laquelle un traitement par des inhibiteurs ECA, des antagonistes AT-1 et/ou des antagonistes du récepteur de l'endothéline est indiqué.

5. Utilisation selon une ou plusieurs des revendications 1 à 4, dans laquelle au moins un inhibiteur de protéasome étant administré à un patient à un dosage d'environ 0,5 µg/kg de poids corporel jusqu'à environ 0,5 mg/kg de poids corporel, de préférence d'environ 1 µg/kg de poids corporel jusqu'à 0,1 mg/kg de poids corporel, de préférence d'environ 0,01 mg/kg de poids corporel jusqu'à 0,1 mg/kg environ de poids corporel.

6. Utilisation selon une ou plusieurs des revendications 1 à 5, **caractérisée en ce que** les maladies fibrotiques sont des maladies fibrotiques d'organes, qui concernent de préférence les poumons, le foie, la peau, les articulations, le squelette et/ou des glandes, en particulier des maladies du système cardiovasculaire.

7. Utilisation selon l'une ou plusieurs des revendications 1 à 6, **caractérisée en ce que** l'inhibiteur de protéasome est un composé organique de faible poids moléculaire ou un composé de biologie moléculaire.

8. Utilisation selon la revendication 7, **caractérisée en ce que** l'inhibiteur de protéasome est un aldéhyde peptidique, un boronate peptidique, une époxycétone peptidique, un lactacystine, une peptide-alpha-cétoaldéhyde, un alpha-cétoamide, un peptide d'indanone, un polyalkylènaldéhyde, un polyphénol, en particulier un catéchine-3-gallate, un acide nucléique dirigé contre au moins un composant du système protéasomique et/ou un anticorps ou sa partie de liaison active ou un dérivé de celui-ci dirigé au moins contre un composant du système protéasomique.

9. Utilisation selon l'une des revendications 7 ou 8, **caractérisée en ce que** l'inhibiteur de protéasome correspond à Z-Leu-Leu-Leu-al (MG132), Z-Ile-Glu(OtNu)-Ala-Leu-al (PSI), CEP1612, pyrazylcarbonyle-Phe-Leu-boronate (PS-341), dansyl-Phe-Leu-boronate (DLFB), morpholinonaphtylalanine-Leu-boronate (MG273), NIP-Leu₃-vinylsulfone (NLVS), Tyr-Leu₃-VS, NIP-Leu-Leu-Asn-VS, Ada-Tyr-Ahx₃-Leu₃-VS, Ada-lys(Bio)-Ahx₃-Leu₃-VS Ac(Me)-Ile-Ile-Thr-Leu-EX (epoxomicine), dihydroéponemycine, lactacystine, clasto-lactacystine-beta-lactone (Omuralide), PS-519, Ae-Leu-Leu-Nle-al (ALLN), 3,4-dichloroisocoumarine (DCI), 4-(2-aminoéthyle)-benzolsulfonylfluorure (Pefablock SC), TMC-95A, gliotoxine, (-)epigallocatéchine-3-gallate (EGCG), ritonavir, lovastatine, aclacinomicine A (aclarubicine), cyclosporine, ARN antisens ou ARN double brin (ARNdb) dirigé contre une séquence codant pour le protéasome, un oligonucléotide constituant un triplex dirigé contre une séquence codant pour un protéasome et/ou une construction knock-out dirigée contre une séquence codant pour un protéasome, où Z est un groupe benzyloxycarbonyle, al un groupe aldéhyde, VS un groupe vinylsulfone, NIP un groupe 3-nitro-4-hydroxy-5-iodophénylacétate et Bio un groupe biotine.
